# EUROPÄISCHE PATENTSCHRIFT

(11) **EP 2 399 539 B1**
(45) Veröffentlichungstag und Bekanntmachung des Hinweises auf die Patenterteilung: **05.11.2014**
(21) Anmeldenummer: 11169475.8
(22) Anmeldetag: 10.06.2011
(51) Int. Cl.: A61B 19/08, A61G 15/10, A61B 17/00, A61B 19/00

(54) **Wegwerfbare Behandlungsstuhlabdeckung im medizinischen Bereich**
Disposable treatment chair covering for medical uses
Recouvrement de siège de traitement jetable dans le domaine médical

(30) Priorität: 17.06.2010 DE 102010024911
(43) Veröffentlichungstag der Anmeldung: 28.12.2011
(73) Patentinhaber: Paul Hartmann AG, 89522 Heidenheim (DE)
(72) Erfinder: Hermandung, Elisabeth, 56355 Nastätten (DE); Niketic, Tatjana, 89522 Heidenheim (DE); Schimek, Gabriele, 73460 Hüttlingen (DE)
(74) Vertreter: DREISS Patentanwälte PartG mbB

(56) Entgegenhaltungen:
- EP-A2- 0 260 182
- GB-A- 944 187
- US-A1- 2005 264 056
- US-A1- 2009 050 162
- US-A1- 2010 140 994

## Beschreibung

Die Erfindung betrifft eine wegwerfbare Behandlungsstuhlabdeckung für den einmaligen Gebrauch für einen Behandlungsstuhl im medizinischen Bereich, insbesondere für einen OP-Stuhl in einem Operationssaal. Ein solcher Behandlungsstuhl weist eine Sitzfläche, eine Rückenlehne und zwei seitliche Armauflagen auf. Er dient bestimmungsgemäß und ausschließlich der im medizinischen Bereich behandelnden Person, insbesondere dem Chirurgen während der Ausführung einer Operation, wobei die behandelnde Person auf dem Behandlungsstuhl Platz nimmt und gegebenenfalls die zwei seitlichen Armauflagen des Behandlungsstuhls zum Abstützen der Unterarme oder der Hand benutzt. Hierfür sind an einem Behandlungsstuhl der hier in Rede stehenden Art typischerweise umfangreiche Verstellmöglichkeiten für die flächenhaft erstreckten Armauflagen vorgesehen. Diese Armauflagen sind typischerweise höhenverstellbar, in einer horizontalen Ebene drehbar oder gar schwenkbar hierzu und weiter insbesondere nach hinten oder vorn oder zu den Seiten hin verstellbar.

Aus der US 2009/0050162 sind haubenartige Abdeckmittel für die seitlichen Armauflagen eines Behandlungsstuhls bekannt. Auch aus US 2007/0246980 A1 ist ein wegwerfbares Abdeckmittel bekannt, welches spezifisch an die Form der Armauflage eines Behandlungsstuhls angepasst ist.

Des Weiteren gibt es gattungsfremde Abdeckhauben für Sitzmöbel, wie Sessel oder Stühle, die jedoch mit einer Behandlungsstuhlabdeckung der hier in Rede stehenden Art im medizinischen Bereich wenig gemein haben und entsprechend ganz anderer Anforderungen hergestellt und ausgebildet sind.

US-A-2010/0140994 (Basis für den Oberbegriff des Anspruchs 1) zeigt eine Abdeckung für eine Vielzahl von Stühlen oder Sitzen, mit den Merkmalen des Oberbegriffs des Anspruchs 1, wobei die Verwendung im medizinischen Bereich nicht explizit erwähnt ist. US-A-2005/0264056 A1 zeigt eine Abdeckung für einen Kinderstuhl mit einem integrierten Gürtel zur Lagesicherung des Kinds.

Der vorliegenden Erfindung liegt die Aufgabe zugrunde, eine auf einfache und wirtschaftliche Weise herstellbare Behandlungsstuhlabdeckung zu schaffen, die, insbesondere im sterilen Bereich eines Operationssaals, einfach zu handhaben ist und mittels derer ein Behandlungsstuhl besser und wirksamer abdeckbar ist, als dies seither der Fall war.

Diese Aufgabe wird durch eine wegwerfbare Behandlungsstuhlabdeckung mit den Merkmalen des Anspruchs 1 gelöst. Danach ist die Abdeckung aus flächenhaften zusammenhängenden Abschnitten eines Flachmaterials gefertigt, die insbesondere aneinandergefügt sind, und herstellerseitig in eine gefaltete, zum bestimmungsgemäßen Gebrauch entfaltbare Konfiguration gebracht ist, wobei die Abdeckung einen ersten der Sitzfläche zugeordneten Abschnitt, einen zweiten der Rückenlehne zugeordneten Abschnitt, und zwei den Armauflagen zugeordnete Seitenabschnitte aufweist, die jeweils spielbehaftet eine Armauflage überfangen, indem sie eine U-Form bilden, die an einer vorderen Stirnseite entlang einer Fügelinie geschlossen ist und an einer gegenüberliegenden hinteren Seite und an einer unteren Seite offen ist. Ferner ist ein jeweiliger Seitenabschnitt auf die spätere Sichtseite des ersten der Sitzfläche zugeordneten Abschnitts und je nach Länge gegebenenfalls auch auf die spätere Sichtseite des zweiten der Rückenlehne zugeordneten Abschnitts eingeschlagen.

Die erfindungsgemäße Abdeckung stellt also ein zusammenhängendes Flächengebilde dar, mittels dessen im Wesentlichen alle Komponenten des Behandlungsstuhls überfangen werden; es werden also nicht einzelne voneinander separate Abdeckhauben für Armauflagen, Rücken- oder Sitzflächen verwandt. Die Abdeckung ist dabei auch derart großzügig in Bezug auf die Abmessungen typischer Behandlungsstühle konfiguriert, dass sie hinreichendes Übermaß aufweist, und jegliche Behandlungsstuhlsituation, und zwar gerade unter Berücksichtigung der verstellbaren Armauflagen adaptieren kann. Dabei kommt gerade der großzügigen Ausbildung der den Armauflagen zugeordneten Seitenabschnitte eine große Bedeutung zu. Sie sind von ihrer flächenhaften Ausladung deutlichst überdimensioniert. Dadurch dass sie mit einer vorderen geschlossenen Stirnseite ausgebildet sind, jedoch nach hinten und nach unten offen sind, lassen sich die Seitenabschnitte sehr einfach und bedienerfreundlich von vorn über die Armauflagen des Behandlungsstuhls stülpen, wobei sie im Bereich des Scheitels der U-Form auf der Oberseite der Armauflagen ruhen. Da die Armauflagen auf ihrer der Stirnseite gegenüberliegenden hinteren Seite und unten offen sind, also die Bereiche der Seitenabschnitte dort nicht miteinander gefügt sind, kann jederzeit Zugang oder Eingriff zu den Verstellmitteln der Armauflagen des Behandlungsstuhls genommen werden, um während der Behandlung die Konfiguration des Behandlungsstuhls, insbesondere die Anordnung der Armauflagen, zu verändern. Aufgrund der spielbehafteten Überfangung der Armauflagen durch die Abdeckung wird eine Verstellung der Armauflagen durch die Abdeckung auch nicht behindert.

In weiterer Ausbildung des Erfindungsgedankens erweist es sich als vorteilhaft, wenn die wegwerfbare Abdeckung eine von dem zweiten Abschnitt gebildete Tasche aufweist, die von oben über die Rückenlehne des Behandlungsstuhls stülpbar ist (Anspruch 2). Diese Tasche kann in bevorzugter Weise durch Umfalten des zweiten Abschnitts auf sich selbst gebildet werden. Zweckmäßigerweise erfolgt die Umfaltung um eine in Querrichtung verlaufende Achse, wobei der umgefaltete Bereich dann in an sich beliebiger Weise auf sich selbst gefügt werden kann, vorzugsweise entlang einer in der Längsrichtung verlaufenden Fügelinie, jeweils links und rechts außen.

Die Tasche weist vorzugsweise eine Erstreckung in Längsrichtung von wenigstens 10 cm, insbesondere wenigstens 20 cm, insbesondere höchstens 50 cm, insbesondere höchstens 40 cm auf.

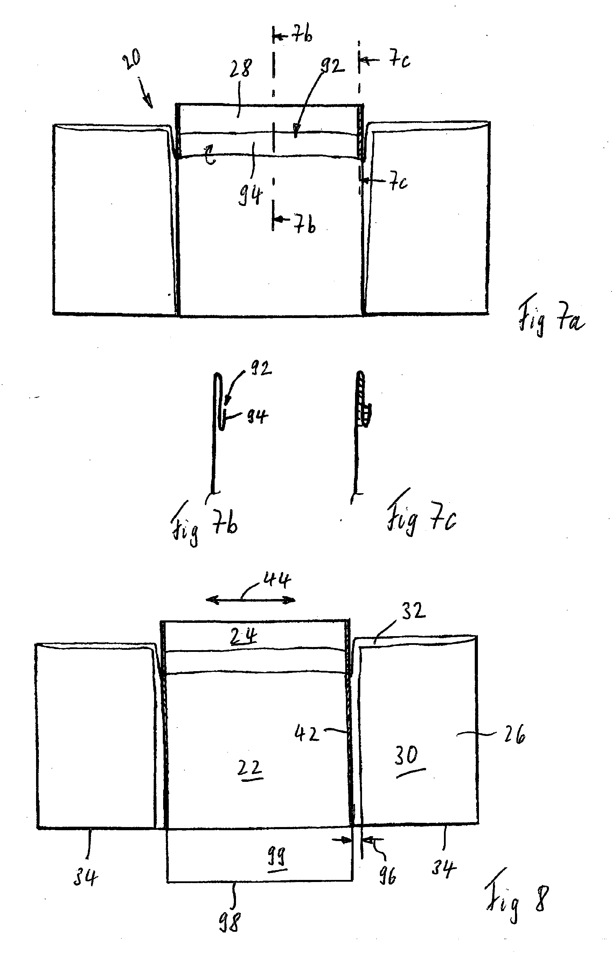

Es erweist sich als vorteilhaft, wenn, insbesondere an der Tasche, eine Eingriffsfalte oder ein zusätzliches manuell ergreifbares Mittel als Handhabungshilfe vorgesehen ist (Anspruch 3). Dies ist in Figuren 7a-c dargestellt. Die Eingriffsfalte ermöglicht eine leichtere und sichere Handhabung der Abdeckung, vor allem beim Überstülpen der Tasche über die Rückenlehne. Durch das Eingreifen in die Eingriffsfalte und damit die Vermeidung von Kontakt mit der an sich unsterilen Stuhllehne wird die Sterilität der Hände bzw. Handschuhe des medizinischen Personals bewahrt.

Diese Eingriffsfalte kann in bevorzugter Weise durch Umfalten eines der Öffnung der Tasche nächstgelegenen Abschnitts entlang einer Querachse nach außen auf sich selbst gebildet werden.
Die Erstreckung der Eingriffsfalte in Längsrichtung beträgt vorzugsweise mindestens 1/5, insbesondere mindestens 1/4 , insbesondere höchstens 1/3 der Erstreckung der Tasche in Längsrichtung, vorzugsweise 3 - 10 cm, weiter vorzugsweise 4 - 8 cm.

Die Fixierung der Eingriffsfalte erfolgt vorzugsweise mittels Ultraschall, Kleber oder Fadennaht.

Diese weitere Eingriffsfalte ist in ihrer Faltung vorzugsweise mittels und entlang der bei der Bildung der Tasche eingebrachten in Längsrichtung verlaufenden Fügelinien fixiert (siehe beispielhaft Figur 7c).

In diesem Zusammenhang sei darauf hingewiesen, dass das Fügen von Abschnitten oder Bereichen der hier in Rede stehenden wegwerfbaren Abdeckung bzw. der die Abdeckung bildenden Flachmaterialien auf an sich beliebige Weise erfolgen kann. Zweckmäßigerweise werden beispielsweise durch Ultraschall oder Temperatur erzeugte durchgehende oder gerasterte, also unterbrochene, Schweißlinien oder Siegellinien verwendet. Es wäre aber auch der alleinige oder zusätzliche Einsatz von Haftvermittlern denkbar. Ebenso ist das Fügen durch Einsatz von Fadennähten denkbar.

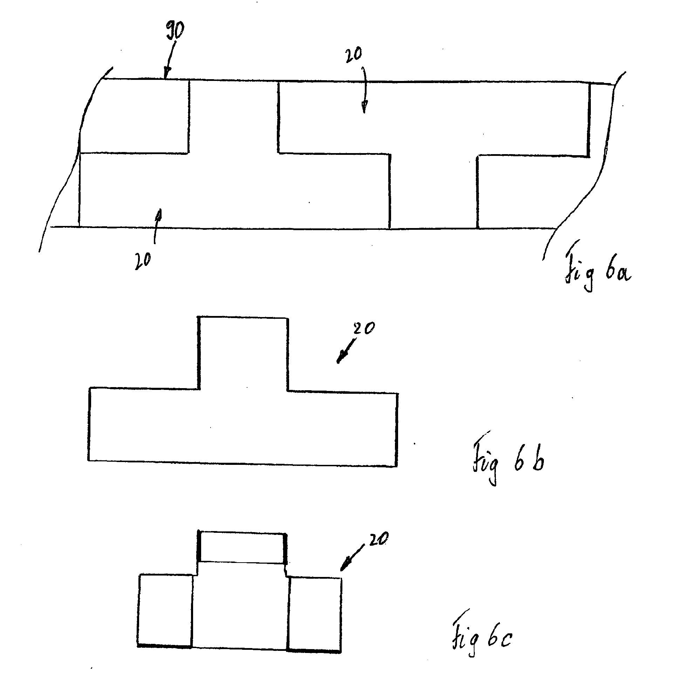

Grundsätzlich wäre es denkbar, dass die Abdeckung aus einem einstückigen Flachmaterial gefertigt ist. Die einstückigen Flachmaterialien können vorzugsweise aus einer Materialbahn durch eine doppelreihige, versetzte Anordnung der auszuschneidenden einstückigen Flachmaterialien gewonnen werden, so wie dies beispielhaft in Figur 6a skizziert ist. Um den bei der Herstellung der Abdeckung anfallenden Schnittabfall zu minimieren, erweist es sich indessen als vorteilhaft, wenn die Seitenabschnitte als separate Abschnitte an den ersten der Sitzfläche zugeordneten Abschnitt der Abdeckung angefügt sind (Anspruch 4). Auch eine zusätzliche Anfügung an wenigstens einen Teil des zweiten der Rückenlehne zugeordneten Abschnitts ist denkbar.

Die Fügelinien der Seitenabschnitte an den ersten und/oder zweiten Abschnitt sind vorteilhaft, indem diese auch als Positionierungshilfe beim Anlegen der Abdeckung an den Stuhl dienen.

Es erweist sich als vorteilhaft, wenn die genannten Seitenabschnitte in einem der vorderen Stirnseite gegenüberliegenden hinteren Bereich nicht an den zweiten der Rückenlehne zugeordneten Abschnitt oder an den ersten der Sitzfläche zugeordneten Abschnitt angebunden sind, insbesondere dass die Seitenabschnitte über wenigstens 7,5 cm, weiter insbesondere über wenigstens 10 cm, weiter insbesondere über wenigstens 15 cm, aber insbesondere über höchstens 30 cm, weiter insbesondere über höchstens 20 cm Längserstreckung nicht an den zweiten der Rückenlehne zugeordneten Abschnitt oder an den ersten der Sitzfläche zugeordneten Abschnitt angebunden sind (Anspruch 5). Durch diese Maßnahme sind die den Armlehnen zugeordneten Seitenabschnitte in ihrem beim bestimmungsgemäßen Gebrauch nach oben erstreckten Bereich freier, so dass sich beim bestimmungsgemäßen Gebrauch und insbesondere beim Verstellen der Armauflagen keine Zugspannungen innerhalb der Abdeckung bilden. Dessen ungeachtet kann eine Umfaltung von Randbereichen bei den Seitenabschnitten vorgesehen werden.

Bei der erfindungsgemäßen Abdeckung können der erste der Sitzfläche zugeordnete Abschnitt und der zweite der Rückenlehne zugeordnete Abschnitt gewissermaßen ununterscheidbar einstückig ineinander übergehen (Anspruch 6); es ist also nicht zwingend eine sitzflächenspezifische oder rückenlehnenspezifische Konfiguration erforderlich. Daher erweist es sich herstellungstechnisch als vorteilhaft, wenn die Abdeckung aus drei vormals separaten Flachmaterialabschnitten, also einem den ersten und den zweiten Abschnitt der Abdeckung bildenden Flachmaterialabschnitt und je einem die beiden Seitenabschnitte bildenden Flachmaterialabschnitt ausgebildet ist.

Die geschlossene vordere Stirnseite des jeweiligen Seitenabschnitts kann vorteilhafterweise kantenfortlaufend in die vordere Kante des ersten der Sitzfläche zugeordneten Abschnitts übergehen. Diese Ausführung ist herstellungstechnisch einfach zu realisieren. Es ist jedoch auch denkbar, dass die vordere Stirnkante des jeweiligen Seitenabschnitts und die vordere Kante des ersten Abschnitts versetzt angeordnet sind, derart dass der erste Abschnitt in Längsrichtung vorsteht, insbesondere indem dort ein zusätzlicher Materialabschnitt angefügt ist (Anspruch 7).

Mit diesem Materialabschnitt kann vorteilhaft ein unterhalb der Sitzfläche angeordneter unterer Bereich des Behandlungsstuhls abgedeckt werden.

Nach einem weiteren Gedanken erweist es sich als vorteilhaft, wenn in einem direkt oberhalb der Armauflagen zu liegen kommenden Bereich der Seitenabschnitte keine Materialübergänge, Fügelinien oder -nähte oder sonstige Unstetigkeiten vorgesehen sind (Anspruch 8). Somit wird die behandelnde Person beim Abstützen der Arme oder Hände auf der Armauflage nicht behindert oder irritiert.

Hinsichtlich der Dimensionierung der erfindungsgemäßen Behandlungsstuhlabdeckung erweist es sich als vorteilhaft, wenn ihre Erstreckung im eben ausgebreiteten Zustand entlang einer Längsrichtung 100 - 150 cm, insbesondere 100 - 140 cm, insbesondere 110 - 130 cm beträgt und/oder die Erstreckung des ersten und des zweiten Abschnitts in einer Querrichtung 80 - 140 cm, insbesondere 80 - 130 cm, insbesondere 80 - 120 cm, insbesondere 80 - 110 cm beträgt und/oder dass die Erstreckung der Seitenabschnitte in der Längsrichtung 80 - 130 cm, insbesondere 90 - 120 cm, insbesondere 90 - 110 cm beträgt und/oder die Erstreckung der Seitenabschnitte ausgehend von dem ersten Abschnitt und/oder dem zweiten Abschnitt in der Querrichtung jeweils 50 - 100 cm, insbesondere 60 - 90 cm, insbesondere 60 - 80 cm beträgt.

Im Hinblick auf eine maschinelle Fertigung der erfindungsgemäßen Behandlungsstuhlabdeckung erweist es sich als vorteilhaft, wenn die Fügung der Seitenabschnitte an den ersten Abschnitt und gegebenenfalls an den zweiten Abschnitt ausschließlich entlang von in Längsrichtung erstreckten Fügelinien hergestellt ist. Des Weiteren könnte es sich als vorteilhaft erweisen, wenn bei der Herstellung der erfindungsgemäßen Behandlungsstuhlabdeckung der Fügevorgang stets in Längsrichtung, also in der Maschinenrichtung, erfolgt, und zwar auch beim Fügen der aufeinander gefalteten Teilabschnitte der Seitenabschnitte, welche dann die geschlossene vordere beim Gebrauch vertikal erstreckte Stirnseite bilden. Solchenfalls müsste eine solche Fügung der die jeweiligen Seitenabschnitte bildenden Flachmaterialien in einem der Anfügung der Seitenabschnitte an den ersten bzw. zweiten Abschnitt der Abdeckung vorausgehenden Verfahrensschritt erfolgen, währenddessen die Flachmaterialien derart gefördert werden, dass die intendierte Fügelinie in Maschinenrichtung verläuft.

Nach einem weiteren Erfindungsgedanken ist ein jeweiliger Seitenabschnitt dadurch gebildet, dass ein Flachmaterialabschnitt um eine in der Längsrichtung verlaufende Achse auf sich selbst gefaltet ist und die aufeinander gefalteten Teilabschnitte entlang eines die vordere Stirnseite der Abdeckung bildenden und in der Querrichtung verlaufenden Rands miteinander gefügt sind und dass ein in der Längsrichtung verlaufender Rand eines der aufeinander gefalteten Teilabschnitte mit dem ersten der Sitzfläche zugeordneten Abschnitt gefügt ist (Anspruch 9).

Es erweist sich als vorteilhaft, wenn die um eine in Längsrichtung verlaufende Achse aufeinander gefalteten Teilabschnitte nicht dieselbe Quererstreckung aufweisen, also in Querrichtung nicht kongruent sind. Vorteilhafterweise weist der für die Fügung an den ersten der Sitzfläche und/oder an zweiten der Rückenlehne zugeordneten Abschnitt vorgesehene Teilabschnitt eine größere Quererstreckung auf, derart, dass dieser Teilabschnitt den anderen Teilabschnitt überragt. Dies ist in Figur 8 beispielhaft dargestellt (siehe Bezugszeichen 96). Damit ist die insbesondere maschinelle Fügung in vorteilhafter Weise leichter und das Risiko einer unbeabsichtigten Fügung des weiteren Teilabschnitts an den ersten und/oder zweiten Abschnitt ist vermindert. Nach diesem weiteren Gedanken wird also Schutz beansprucht dafür, dass ein äußerer Teilabschnitt zweier sich parallel zueinander erstreckender Teilabschnitte der Seitenabschnitte, welcher nicht an den ersten der Sitzfläche zugeordneten Abschnitt angebunden ist, in Querrichtung kürzer erstreckt ist als der innere Teilabschnitt, der an den ersten der Sitzfläche zugeordneten Abschnitt angebunden ist.

Die vorstehend erwähnte Längsrichtung und Querrichtung bezieht sich jeweils auf die fertig hergestellte wegwerfbare Behandlungsstuhlabdeckung im eben ausgebreiteten Zustand. Es ist zwar möglich, dass die Längsrichtung der Abdeckung - wie vorausgehend als vorteilhaft angedeutet - während der Herstellung stets in Maschinenrichtung verläuft; dies ist jedoch nicht zwingend, insbesondere können Flachmaterialien und Flachmaterialabschnitte während der Herstellung in der Maschine insbesondere um 90° gedreht werden. Auf diese Weise ist es möglich, Fügeverbindungen vorzugsweise in der Maschinenrichtung zu realisieren.

Mit der erfindungsgemäßen Abdeckung wird auch eine für eine Vielfalt an Ausführungen von Behandlungsstühlen einsetzbare Abdeckung bereitgestellt. Wie vorstehend dargestellt zeichnet sich die erfindungsgemäße Abdeckung durch eine spielbehaftete Überfangung der Armauflagen aus. Dennoch kann es auch von Vorteil sein, abhängig von der Größe des Behandlungsstuhls bzw. von dessen im Einsatz individuell eingestellten Positionen, wie beispielsweise hoch oder niedrig eingestellter Sitzfläche oder in der Waagrechten nach vorn oder hinten verschobenen Armauflagen, die Abdeckung im drapierten aufgezogenen Zustand lösbar zu fixieren. Vorteilhafterweise ist die Abdeckung hierfür mit einem lösbar haftend wirkenden Fixiersystem ausgestattet (Anspruch 10).

Dafür ist vorzugsweise vorgesehen, die Seitenabschnitte in einem der vorderen geschlossenen Stirnkante gegenüberliegenden hinteren Bereich mit einer ersten Fixierkomponente auszustatten (Anspruch 11). Vorzugsweise ist die erste Fixierkomponente als Kletthakenkomponente ausgebildet. Der der Rückenlehne zugeordnete zweite Abschnitt ist vorzugsweise auf dem die Tasche bildenden hinteren Teilstück mit einer zweiten Fixierkomponenten ausgestattet. Vorzugsweise ist die zweite Fixierkomponente als Klettflauschkomponente ausgebildet. Die zweite Fixierkomponente ist vorzugsweise streifenförmig in Querrichtung angeordnet vorgesehen, um eine individuell bedarfsgerechte Landezone für die erste Fixierkomponente bereitzustellen. Vorzugsweise ist die zweite Fixierkomponente oberhalb der Eingriffsfalte der Tasche des zweiten der Rückenlehne zugeordneten Abschnitts angebracht.

In weiterer Ausbildung der Erfindung erweist es sich als vorteilhaft, wenn bei den Seitenabschnitten (gegenüberliegend zu dem die Stirnseite bildenden gefügten vorderen Rand) eine Umfaltung um eine in der Querrichtung verlaufende Achse auf sich selbst vorgesehen ist (Anspruch 12). Insbesondere ist die Umfaltung wenigstens 5 cm, weiter insbesondere wenigstens 7,5 cm, weiter insbesondere wenigstens 10 cm, weiter insbesondere höchstens 30 cm, weitere insbesondere höchstens 25 cm, weiter insbesondere höchstens 20 cm tief. Auf diese Weise bilden die freien Randbereiche der hinteren offenen Seite der den Armauflagen zugeordneten Seitenabschnitte einen Umschlag, der sich in mehrfacher Hinsicht als vorteilhaft erweisen kann. Er könnte einerseits bei der Handhabung, also beim Entfalten der erfindungsgemäßen Abdeckung eine Handhabungshilfe bilden. Andererseits vermag dieser Umschlag oder diese Umfaltung andere eingefaltete Randbereiche der Abdeckung zu überfangen und dadurch zu schützen und möglicherweise zu halten. Insbesondere kann durch die Umfaltung die am Seitenabschnitt angebrachte erste Fixierkomponente überdeckt und damit vor einer ungewollten zufälligen Fixierung geschützt werden.

Nach einem weiteren Gedanken erweist es sich als vorteilhaft, wenn ein jeweiliger Seitenabschnitt um eine diagonal, also schräg zur Längsrichtung und zur Querrichtung verlaufende Faltachse auf sich selbst nach innen gefaltet ist (Anspruch 13). Die Faltachse verläuft dabei derart, dass eine äußere vordere Ecke (im eben ausgebreiteten Zustand der Abdeckung) des Seitenabschnitts in Richtung auf den ersten oder zweiten Abschnitt der Abdeckung gefaltet wird. Hierbei erweist es sich als vorteilhaft, wenn diese Faltung derart ist, dass sich der gefaltete Bereich unter die vorausgehend erwähnte randseitige Umfaltung erstreckt, also unter die hierdurch gebildete Eingriffsfalte (Anspruch 14).

Nach einem weiteren Erfindungsgedanken wird vorgeschlagen, dass ein jeweiliger Seitenabschnitt um eine im Bereich der Anfügung der Seitenabschnitte an den ersten Abschnitt liegende in Längsrichtung erstreckte Achse auf die spätere Sichtseite des ersten der Sitzfläche zugeordneten Abschnitts eingeschlagen ist (Anspruch 15).

In weiterer Ausbildung dieses Erfindungsgedankens erweist es sich als vorteilhaft, wenn der jeweilige diagonal gefaltete Seitenabschnitt und/oder der jeweilige auf die spätere Sichtseite des ersten der Sitzfläche zugeordneten Abschnitts eingeschlagene Seitenabschnitt durch manuell lösbare Fixiermittel, insbesondere in Form von Haftklebemitteln, insbesondere mit Sollbruchlinien, in der gefalteten Konfiguration gehalten ist (Anspruch 16). Weiter erweist es sich als vorteilhaft, wenn der so gebildete Verbund aus erstem und zweitem Abschnitt und darauf gefalteten Seitenabschnitten weiter um wenigstens

Verbund aus erstem und zweitem Abschnitt und darauf gefalteten Seitenabschnitten weiter um wenigstens drei in Querrichtung verlaufende Faltachsen auf sich selbst gefaltet ist, und zwar vorzugsweise derart, dass ein in Längsrichtung endseitiger Teilabschnitt des zweiten der Rückenlehne zugeordneten Abschnitts oben zu liegen kommt (Anspruch 17). Auf diese Weise wird erreicht, dass ein dem oberen Ende der Rückenlehne des Behandlungsstuhls zugeordneter Bereich der Abdeckung oben angeordnet ist. Ein Benutzer vermag dann beim Entfalten des herstellerseitig gefalteten Bezugs zunächst diesen oben liegenden Teilabschnitt, der wie erwähnt insbesondere eine Tasche oder eine sonstige Fixiermöglichkeit für das obere Ende der Rückenlehne des Behandlungsstuhls umfassen kann, über die Rückenlehne stülpen kann, die typischerweise den höchst gelegenen Punkt des Behandlungsstuhls über dem Boden bildet. Das weitere Entfalten ist dann erleichtert.

In weiterer Ausbildung dieses Erfindungsgedankens erweist es sich als vorteilhaft, wenn die Abdeckung um wenigstens zwei in Querrichtung verlaufende Faltachsen Z-förmig auf sich selbst gefaltet ist und der in Längsrichtung endseitige Teilabschnitt des zweiten, der Rückenlehne zugeordneten Abschnitts wiederum oben zu liegen kommt (Anspruch 18). Durch eine Z-förmige Faltung ist dann das weitere Entfalten kontrolliert möglich. Es ist aber auch denkbar, dass die Abdeckung um wenigstens zwei in Querrichtung verlaufende Faltachsen förmlich aufgerollt ist und der in Längsrichtung endseitige Teilabschnitt des zweiten, der Rückenlehne zugeordneten Abschnitts wiederum oben zu liegen kommt. Durch die aufrollende Faltung ist dann das weitere Entfalten noch weitergehend erleichtert.

Insbesondere nach dem herstellerseitigen Falten um in Querrichtung verlaufende Faltachsen erweist es sich weiter als vorteilhaft, wenn die Abdeckung zusätzlich einmal oder mehrmals um in Längsrichtung verlaufende Faltachsen auf sich selbst gefaltet ist (Anspruch 19). Diejenigen Faltungen werden dann beim Entfalten der Abdeckung zuerst entfaltet.

Nach einem weiteren Erfindungsgedanken hinsichtlich der herstellerseitig vorzunehmenden Faltung der Behandlungsstuhlabdeckung erweist es sich als vorteilhaft, wenn die Seitenabschnitte, insbesondere nach dem Einschlagen auf die spätere Sichtseite des ersten der Sitzfläche zugeordneten Abschnitts, um wenigstens eine in Längsrichtung verlaufende Faltachse auf sich selbst gefaltet sind (Anspruch 20). Insbesondere sind die Seitenabschnitte zumindest um eine weitere in Längsrichtung jedoch weiter innen liegende Faltachse nach außen auf sich selbst umgefaltet.

Die Seitenabschnitte werden solchenfalls vorzugsweise mehrfach auf sich selbst gefaltet, so dass jeder Seitenabschnitt gewissermaßen sein eigenes Faltpaket bildet, wobei nicht ausgeschlossen werden soll, dass sich die Seitenabschnitte mittig im eingeschlagenen Zustand überlappen.

Weiter erweist es sich bei dieser Längsfaltung als vorteilhaft, wenn die wenigstens eine Faltung um die wenigstens eine in Längsrichtung verlaufende Faltachse auch den ersten und zweiten Abschnitt der Behandlungsstuhlabdeckung, die der Sitzfläche und der Rückenlehne zugeordnet sind, erfasst (Anspruch 21). Solchenfalls lässt sich nämlich ein von vornherein insgesamt schmaler bauendes Faltpaket realisieren. Dies bietet den Vorteil, dass eine einzige weitere Längsfaltung, vorzugsweise nach mehreren Querfaltungen, ausreicht, um eine für Handel- und Handhabungszwecke durch den Endverbraucher geeignete Abmessung des Faltpakets zu erreichen.

Insbesondere vorteilhaft ist an einem nach außen gewandten Bereich des so "gestapelt" gefalteten Seitenabschnitts ein manuell lösbares Fixiermittel vorgesehen, um die "gestapelte" Konfiguration lösbar zu fixieren.

Diese derart vorgefaltete Behandlungsstuhlabdeckung wird vorzugsweise, wie voranstehend dargestellt, um in Querrichtung verlaufende Faltachsen gefaltet, und weiter insbesondere nach dem Falten um in Querrichtung verlaufenden Faltachsen vorzugsweise um zumindest eine in Längsrichtung verlaufende Faltachse auf sich selbst gefaltet. Damit wird wiederum eine kompakt gefaltete Konfiguration für die Abgabe an den Handel erreicht.

Gegenstand der Erfindung ist auch ein Behandlungsstuhl im medizinischen Bereich, insbesondere ein OP-Stuhl für die Verwendung in einem Operationssaal, mit einer Sitzfläche, einer Rückenlehne und zwei seitlichen vorzugsweise verstellbaren Armauflagen, der von einer wegwerfbaren Abdeckung der vorausgehend beschriebenen Art überfangen ist.

Die Abdeckung ist für Behandlungsstühle unterschiedlicher Einsatzbereiche denkbar, vor allem in denen die behandelnde Person eine Stützung der Hände oder Unterarme mittels Armauflagen benötigt. Ein derartiger Behandlungsstuhl wird beispielsweise bei mikroskopieunterstützten Operationen eingesetzt.

Die Abdeckung ist vorzugsweise sterilisierbar. Die Abdeckung wird vorzugsweise als sterile Abdeckung bereitgestellt.

Als Material für die Abdeckung wird insbesondere ein- oder mehrlagiges Vliesstoffmaterial, insbesondere in Form von Spinnvlies (S)- und/oder Meltblown (M) Vlieslagen, insbesondere ein Laminat aus einer oder mehreren Spinnvlies (S)- und/oder Meltblown (M) Vlieslagen, insbesondere bevorzugt als SM, SMS, SMMS, SMMMS eingesetzt. Besonders bevorzugt besteht das Vliesstoffmaterial aus Polyethylen und/oder Polypropylen und/oder aus Mischungen davon als Hauptkomponente. Das Vliesstoffmaterial weist vorzugsweise ein Flächengewicht von 10 - 70 g/m², weiter vorzugsweise von 10 - 60 g/m², besonders bevorzugt von 10 - 50 g/m², insbesondere von 10 - 40 g/m², insbesondere 10 - 35 g/m², weiter insbesondere 15 - 35 g/m², weiter insbesondere 20 - 35 g/m² auf.

Die Abdeckung kann alternativ Folienmaterial umfassen oder aus einem solchen bestehen. Vorzugsweise besteht das Folienmaterial aus Polyethylen und/oder Polypropylen und/oder aus Mischungen davon. Das Folienmaterial ist vorzugsweise eine nicht mikroporöse Folie. Die Folie hat ein Flächengewicht von vorzugsweise 15 - 35 g/m², insbesondere 20 - 30 g/m².

Weiter vorzugsweise wird ein Folienmaterial als PE-Film mit einer Dicke von 40, 60 oder 70 µm eingesetzt.

Alternativ kann die Abdeckung auch aus einem Vlies-Folien-Verbund gebildet sein oder einen solchen umfassen, insbesondere mit den voranstehend genannten Vliesstoff- und Folienmaterialien.

Dabei kann die Vliesschicht auf einer oder auf beiden Seiten der Folie vorgesehen sein.

Im Falle eines Vlies-Folien-Verbundes beträgt das Flächengewicht des Vlieses vorzugsweise 10 - 40 g/m², insbesondere 10-35 g/m², weiter insbesondere 15-35 g/m², weiter insbesondere 20-35 g/m² und die Folie hat ein Flächengewicht von vorzugsweise 15 - 35 g/m², insbesondere 20 - 30 g/m².

Die Laminierung des Vlies-Folien-Verbundes kann auf jegliche Art erfolgen, vorzugsweise sind Vlies-Folie mittels Thermobonding und/oder Kleber, insbesondere Hotmelt verbunden.

Im Falle einer Abdeckung mit oder aus Vlies-Folien-Verbund ist die aus Folienmaterial gebildete Oberseite vorzugsweise zum Behandlungsstuhl hin orientiert ausgerichtet.

Vliesstoffe besitzen den Vorteil, neben einer mechanischen Festigkeit, die sie einer Folie verleihen können, taktile Eigenschaften zu besitzen, die insbesondere bei den Seitenabschnitten der Abdeckung, welche über den Armauflagen des Behandlungsstuhles zu liegen kommen, der Abdeckung insbesondere in dem für die Stützung der Hände und/oder Unterarme vorgesehenen Bereich ein rutschfesteres Verhalten für die behandelnde Person ermöglichen.

Das Material der Abdeckung weist vorzugsweise eine Widerstandsfähigkeit gegen Flüssigkeitspenetration (die sogenannte Wassersäule) auf, vorzugsweise eine Wassersäule von mindestens 150 cm, weiter vorzugsweise von 150 - 500 cm, weiter vorzugsweise von 300 - 500 cm auf. Die Wassersäule wird gemessen nach EN 20811, bei einem Gradienten von 60 mbar/ min - gemittelt über 5 Messungen. Eine gewisse Flüssigkeitsundurchlässigkeit ist vorteilhaft, um den Behandlungsstuhl vor während der Behandlung bzw. Operation möglicherweise auftretenden Flüssigkeiten zu schützen, um somit auch aufwendige Reinigungsarbeiten zu minimieren.

Vorzugsweise weist das Material der Abdeckung einen Linting -Koeffizienten kleiner 3, weiter vorzugsweise kleiner 2,7, insbesondere kleiner 2,5, insbesondere kleiner 2,2 auf. Linting ist die Tendenz eines Textils oder Vliesstoffes Eigenmaterial in Form von Bruchstücken während des Gebrauchs abzugeben. Für einen Vliesstoff bedeutet Linting die Abgabe von Partikeln wie Faserbruchstücke oder sonstige Komponenten, die zur Herstellung eines Vliesstoffes Verwendung finden, während des Gebrauchs. Aus Sterilitätsgründen und Gründen der Verunreinigung des Operationsbereiches und auch wenn es um die Reinheit der Arbeitsatmosphäre geht sollten möglichst wenig Partikel freigesetzt werden. Insbesondere im Bereich der Seitenabschnitte der Abdeckung sollte lintingarmes Material eingesetzt werden.

Die Feststellung des Linting-Koeffizienten erfolgt nach dem internationalen Standard ISO 9073-10. Zur Messung wird ein Gerät Gelbo Flex 5000 der Firma Instrument Marketing Services/Fairfield sowie ein Counter LS 31C der Firma SFP eingesetzt.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen wegwerfbaren Behandlungsstuhlabdeckung.

In der Zeichnung zeigt:
- Figur 1: eine perspektivische Ansicht eines typischen Behandlungsstuhls im medizinischen Bereich;
- Figur 2: einen mit einer erfindungsgemäßen Behandlungsstuhlabdeckung überfangenen Behandlungsstuhl nach Figur 1;
- Figur 3: eine Draufsicht auf die erfindungsgemäße Behandlungsstuhlabdeckung im eben ausgebreiteten Zustand;
- Figuren 4a bis i: einen herstellerseitig vorgenommenen Faltvorgang der erfindungsgemäßen Behandlungsstuhlabdeckung vor der Verpackung des Produkts zur Abgabe an den Letztverbraucher;
- Figuren 5a bis h: einen herstellerseitig vorgenommenen alternativen Faltvorgang;
- Figuren 6a bis c: die einstückige Herstellung der Behandlungsstuhlabdeckung;
- Figuren 7a bis c: die Ausbildung einer Handhabungshilfe;
- Figur 8: weitere vorteilhafte Detailmerkmale der erfindungsgemäßen Behandlungsstuhlabdeckung und
- Figuren 9 und 10: die lösbare Fixierung der Behandlungsstuhlabdeckung im auf einen Behandlungsstuhl aufgezogenen Zustand.

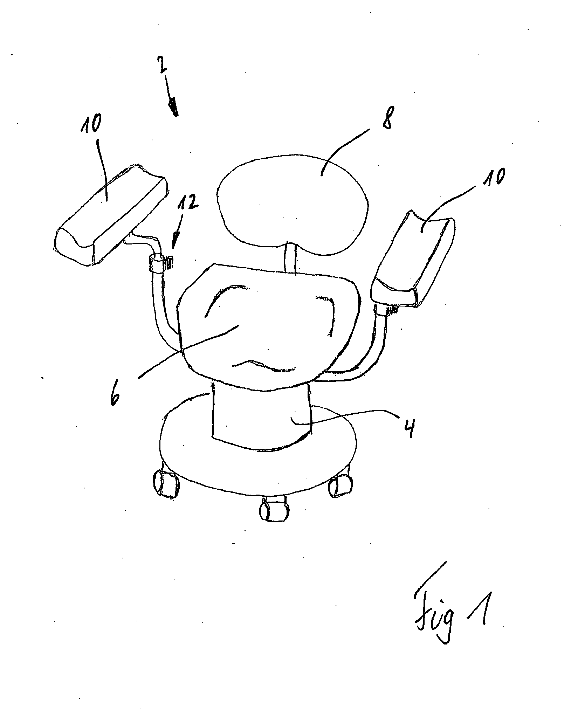

Figur 1 zeigt einen Behandlungsstuhl 2 im medizinischen Bereich, insbesondere einen OP-Stuhl. Der Behandlungsstuhl 2 weist eine auf mehreren Rollen verfahrbare Basiskonstruktion 4, eine Sitzfläche 6 und eine Rückenlehne 8 sowie zwei seitliche Armauflagen 10 auf. Die beidseitigen Armauflagen 10 sind höhenverstellbar und verschwenkbar und gegebenenfalls neigbar über einen nur schematisch skizzierten Verstellmechanismus 12 vorgesehen. Auf diese Weise können die Armauflagen individuell auf die Bedürfnisse der behandelnden Person, insbesondere des Chirurgen, und auf die spezifischen Anforderungen der auszuführenden medizinischen Behandlung eingestellt werden.

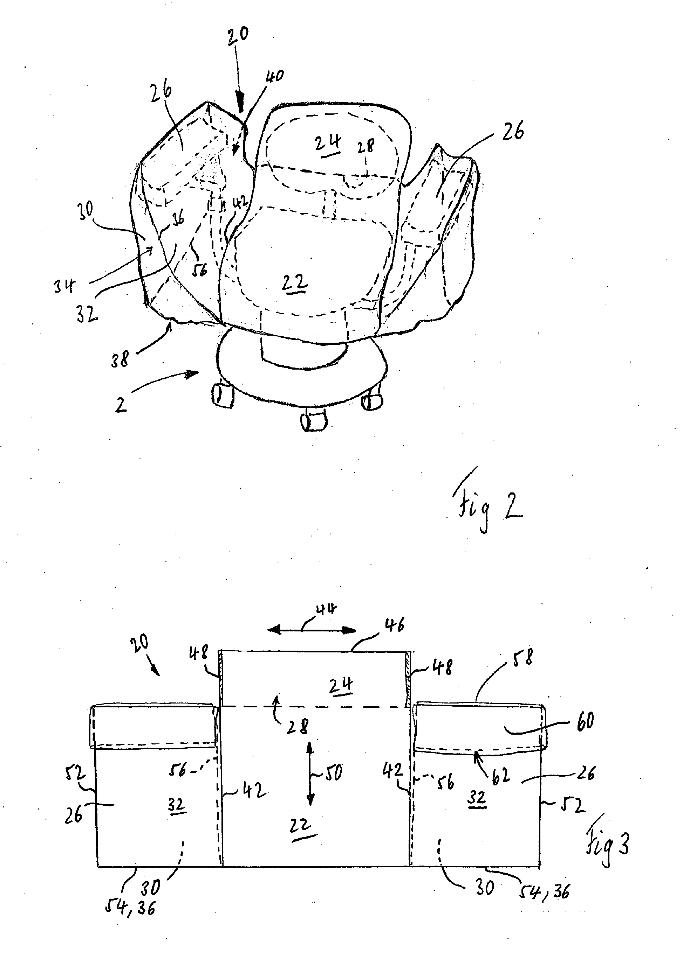

Figur 2 zeigt den Behandlungsstuhl 2 sowie eine den Behandlungsstuhl 2 überfangende erfindungsgemäße Behandlungsstuhlabdeckung 20. Die erfindungsgemäße Abdeckung 20 ist aus flächenhaften und zusammenhängenden Abschnitten eines Flachmaterials gefertigt. Sie umfasst einen ersten der Sitzfläche 6 des Behandlungsstuhls 2 zugeordneten Abschnitt 22, einen zweiten der Rückenlehne 8 des Behandlungsstuhls 2 zugeordneten Abschnitt 24 sowie zwei den Armauflagen 10 zugeordnete Seitenabschnitte 26. Die erfindungsgemäße Abdeckung 20 überfängt im Wesentlichen sämtliche Komponenten des Behandlungsstuhls 2. Die behandelnde Person vermag auf der Abdeckung 20 im Wesentlichen unbehindert Platz zu nehmen, ohne mit den Armen (mit Ausnahme der Beine) in Kontakt mit Teilen oder Komponenten des Behandlungsstuhls 2 zu kommen, die sämtlich von der Abdeckung 20 spielbehaftet überfangen sind.

Man erkennt, dass bei dem der Rückenlehne 8 zugeordneten Abschnitt 24 der Abdeckung 20 eine Tasche 28 ausgebildet ist, und zwar indem der zweite Abschnitt 24 auf sich selbst umgefaltet ist. Mit dieser Tasche 28 wird die Abdeckung 20 über die Rückenlehne 8 gestülpt und ist hierdurch gehalten. Die noch näher zu beschreibenden Seitenabschnitte 26 sind aus auf sich selbst gefalteten Flachmaterialabschnitten gebildet, wobei die aufeinander gefalteten Teilabschnitte U-förmig gefaltet sind, wobei der Scheitel der U-Form entlang der Armauflagen 10 verläuft, so dass ein äußerer Teilabschnitt 30 und ein innerer Teilabschnitt 32 gebildet ist, die entlang einer eine vordere Stirnseite 34 bildendenden Fügelinie 36 miteinander gefügt sind. Unten (Bezugszeichen 38) und hinten (Bezugszeichen 40) sind die Teilabschnitte 30 und 32 nicht miteinander gefügt, so dass die Seitenabschnitte 26 dort offen sind, was das Überstülpen der Seitenabschnitte über die Armlehnen 10 einfach gestaltet. Außerdem ist hierdurch jederzeit ein Zugriff zu den Verstellmechanismen 12 des Behandlungsstuhls 2 möglich. Die Seitenabschnitte 26 sind jeweils über den inneren Teilabschnitt 32 entlang eines seitlichen Längsrands 42 mit dem ersten der Sitzfläche zugeordneten Abschnitt 22 gefügt.

Figur 3 zeigt eine Draufsicht auf die eben ausgebreitete erfindungsgemäße Abdeckung 20. Man erkennt, dass die vorausgehend erwähnte Tasche 28 durch Umfalten des zweiten Abschnitts 24 um eine in Querrichtung 44 erstreckte Faltachse 46 gebildet ist, wobei im Längsrandbereich beidseitig eine Fügeverbindung 48 zur Fixierung der Umfaltung vorgesehen wurde. Wie erwähnt, sind die beidseitigen Seitenabschnitte 26 durch eine Faltung eines Flachmaterials auf sich selbst um eine in Längsrichtung 50 verlaufende Faltachse 52 gebildet. Der innen liegende Teilabschnitt 32 ist dann entlang der Fügelinie 42 an den ersten Abschnitt 22 unlösbar angefügt. Der andere Teilabschnitt 30 ist in der Figur 3 verdeckt durch eine unterbrochene Linie dargestellt. Die aufeinander zu liegen kommenden Teilabschnitte 30 und 32 sind entlang des in Querrichtung 44 verlaufenden Rands 54 miteinander unlösbar gefügt, wodurch die schon im Zusammenhang mit Figur 2 erwähnte Fügelinie 36 gebildet ist, welche im Bereich der vorderen Stirnseite 34 der Seitenabschnitte 26 bei der bestimmungsgemäßen Verwendung der Abdeckung 20 verläuft.

Man erkennt ferner, dass der in Figur 3 verdeckte Teilabschnitt 30 nicht an den ersten Abschnitt 22 angefügt ist, sondern einen freien Rand 56 bildet, der in Figur 2 unten frei endet, so dass die Seitenabschnitte 26 nach unten offen sind. Die Teilabschnitte 30 und 32 sind an ihrem in Längsrichtung 50 dem gefügten Rand 54 gegenüberliegenden Rand 58 ebenfalls nicht miteinander gefügt, so dass die Seitenabschnitte 26 auch hinten offen sind (Bezugszeichen 40 in Figur 2). Man erkennt in Figur 3 dort aber eine Umfaltung 60 der Teilabschnitte 30 und 32 nach außen auf sich selbst, wodurch eine Eingriffsfalte 62 gebildet ist, in welche manuell eingegriffen werden kann oder die lediglich der Aufnahme oder dem Schutz weiterer Bestandteile der Abdeckung dient, was nachfolgend beschrieben werden wird. Die Umfaltung 60 kann vor dem Falten der Seitenabschnitte um die Faltachse 52 vorgenommen werden, was die maschinelle Herstellung erleichtert, oder dies kann danach geschehen.

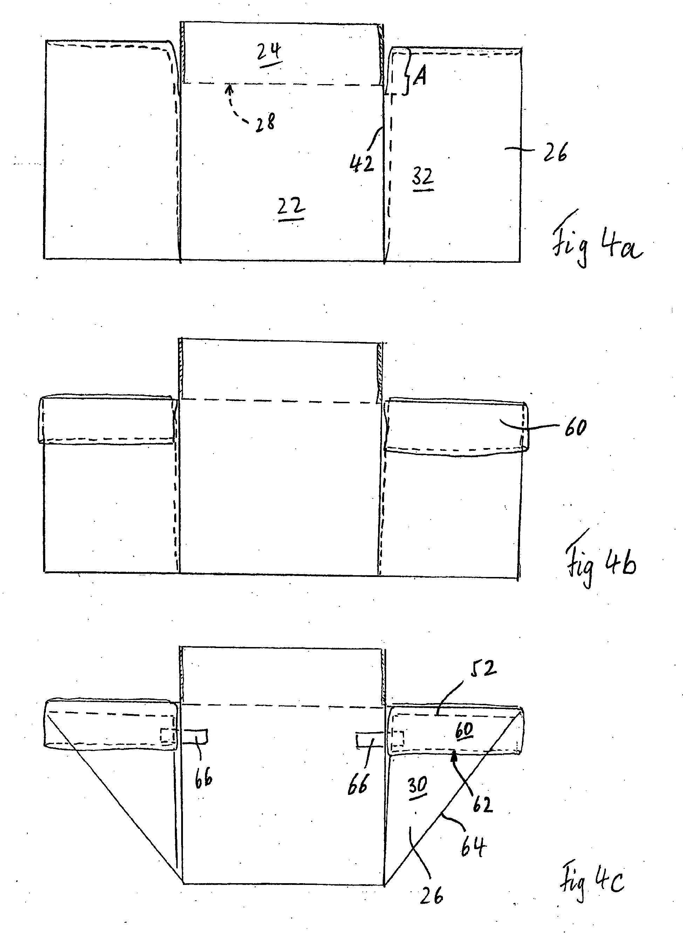

Die Figuren 4a bis i zeigen eine vorteilhafte herstellerseitige Faltung der an sich fertigen Behandlungsstuhlabdeckung 20. Aus Figur 4a ist erkennbar, dass der jeweilige innere Teilabschnitt 32 der Seitenabschnitte 26 nicht über seine gesamte Längserstreckung an den ersten Abschnitt 22 angefügt ist, sondern dass die Fügelinie 42 in einem Abstand A vor dem Längsende des Teilabschnitts 32 endet, was sich als besonders vorteilhaft erweist. Hierdurch ist eine Entkopplung eines Teils der Seitenabschnitte 26 von dem ersten oder zweiten Abschnitt 22, 24 erreicht, die die Handhabung der Abdeckung erleichtert und außerdem eine randseitige Umfaltung 60 bei den Seitenabschnitten 26 ermöglicht, was in Figur 4b (identisch zu Figur 3) dargestellt ist. Figur 4c zeigt dann eine Faltung der Seitenabschnitte 26 um eine schräg zur Längsrichtung 50 und schräg zur Querrichtung 44 verlaufende Faltachse 64, derart, dass die Seitenabschnitte durch Faltung um eine Diagonale flächenmäßig halbiert werden (dies muss aber nicht zwingend der Fall sein). Dabei wird der Seitenabschnitt 26 mit der Faltachse 52 in die von der Umfaltung 60 gebildete Eingriffsfalte 62 hineingeschoben, also von der Umfaltung 60 verdeckt und geschützt. Man erkennt ferner ein in Figur 4c erstmals dargestelltes manuell lösbares Fixiermittel 66, mit dem die Seitenabschnitte 26 so lange in ihrer gefalteten Konfiguration gehalten werden, bis ein Benutzer die Fixiermittel 66 löst. Hierbei kann es sich um an sich beliebige klebende oder mechanisch haftende Fixiermittel, insbesondere Klebetapes mit oder ohne Sollbruchstellen zum Öffnen handeln.

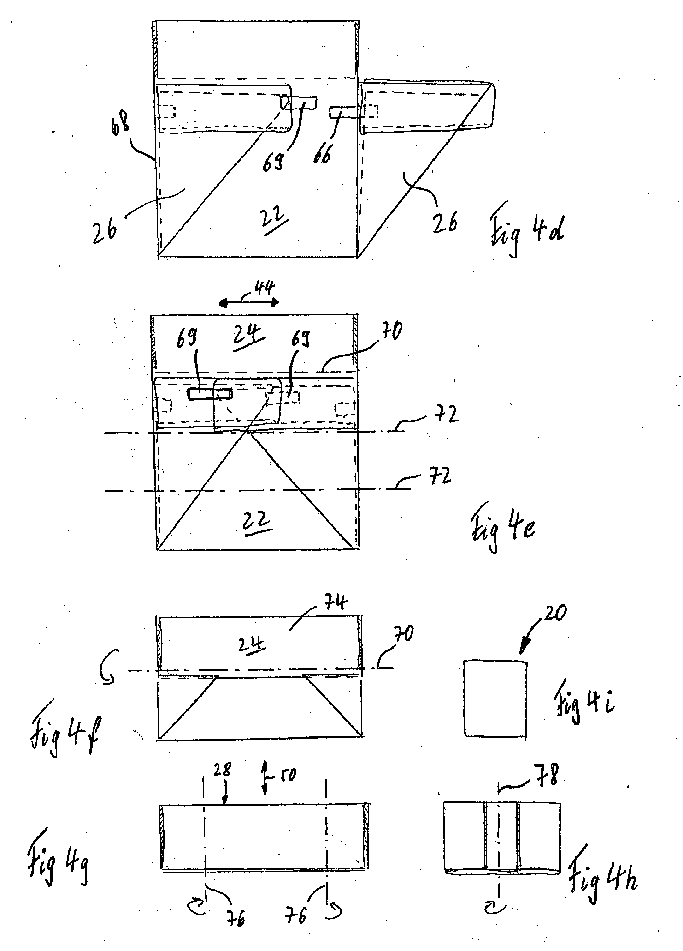

Figur 4d zeigt wie ein jeweiliger Seitenabschnitt 26 um eine im Bereich der Anfügung der Seitenabschnitte 26 an den ersten Abschnitt 22 liegende und in Längsrichtung 50 erstreckte Faltachse 68 auf die spätere Sichtseite des ersten Abschnitts 22 eingeschlagen ist, so dass die in Figur 4e dargestellte Konfiguration erhalten wird. Die eingeschlagenen Seitenabschnitte 26 sind dann durch weitere manuell lösbare Fixiermittel 69 gehalten.

In Figur 4e sind weiter drei in Querrichtung 44 verlaufende Faltachsen 70 bzw. 72 eingezeichnet. Um die Faltachsen 72 wird der erste Abschnitt 22 zusammen mit den auf ihn eingeschlagenen Seitenabschnitte 26 vorzugsweise Z-förmig auf sich selbst gefaltet, so dass die in Figur 4f dargestellte Konfiguration erhalten wird. Daran anschließend wird ein endseitiger Teilabschnitt 74 des zweiten der Rückenlehne zugeordneten Abschnitts 24 um die Faltachse 70 auf die zuvor erwähnte Z-förmig gefaltete Konfiguration gefaltet, so dass die in Figur 4g dargestellte Anordnung erhalten wird. In Figur 4g sind weiter beispielhaft zwei weitere, jedoch in Längsrichtung 50 verlaufende Faltachsen 76 dargestellt, um die eine weitere Faltung in die in Figur 4h dargestellte Konfiguration vorgenommen wird. Schließlich wird die in Figur 4h dargestellte Konfiguration um eine weitere in Längsrichtung 50 verlaufende Faltachse 78 gefaltet, so dass die in Figur 4i dargestellte im Wesentlichen rechteckförmige Konfiguration der gefalteten Abdeckung 20 erhalten wird. Die Entfaltung der erfindungsgemäßen Abdeckung erfolgt in umgekehrter Reihenfolge, also ausgehend von der Konfiguration nach Figur 4i. Die Abdeckung wird dann mit der Tasche 28 über die Rückenlehne 8 des Behandlungsstuhls 2 gestülpt und dann wird in der Entfaltungsreihenfolge weiter entfaltet. Nach dem Lösen der Fixiermittel 66 und 69 können die Seitenabschnitte 26 weiter entfaltet und von vorn über die Armauflagen 10 gestülpt werden. Hierbei wird die Umfaltung 60 entfaltet, was dann die Länge der Seitenabschnitte 26 im Bereich der Armauflagen 10 vergrößert.

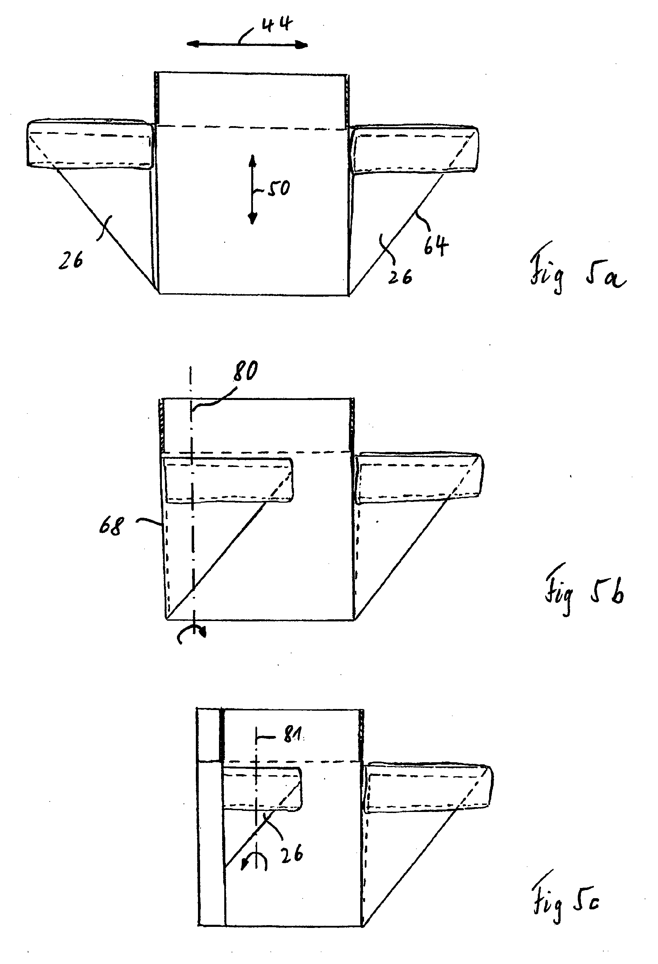

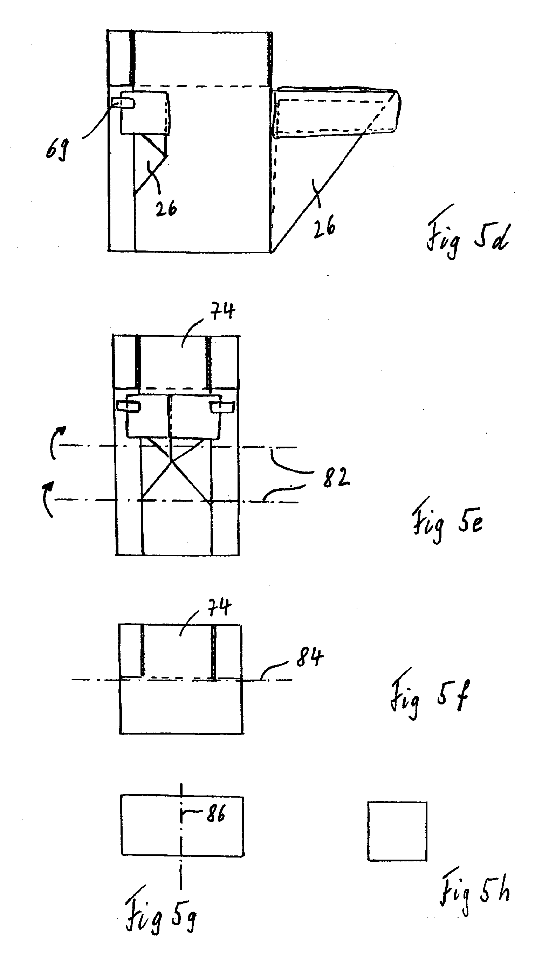

Figuren 5a bis h zeigen eine weitere bevorzugte Faltung der erfindungsgemäßen Behandlungsstuhlabdeckung 20, wobei Figuren 5a und b den Figuren 4c und d entsprechen und die Faltung um die schräg zur Querrichtung 44 verlaufende Faltachse 64 sowie die Einfaltung der Seitenabschnitte 26 um die in Längsrichtung 50 erstreckte Faltachse 68 zeigt, wobei aber manuell lösbare Fixiermittel zunächst nicht verwendet werden. Figur 5b zeigt eine weitere in Längsrichtung 50 verlaufende Faltachse 80, um welche die Behandlungsstuhlabdeckung mitsamt dem eingeschlagenen Seitenabschnitt 26 weiter auf sich selbst nach innen eingeschlagen ist, so dass die in Figur 5c dargestellte Konfiguration erhalten wird. In Figur 5c ist eine weitere in Längsrichtung 50 jedoch weiter innen liegende Faltachse 81 dargestellt, um welche diesmal nur der eingeschlagene Seitenabschnitt 26 nach außen auf sich selbst umgefaltet ist, so dass die in Figur 5d dargestellte Konfiguration erhalten wird. An dem nach außen gewandten Bereich des so "gestapelt" gefalteten Seitenabschnitts 26 ist schließlich ein manuell lösbares Fixiermittel 69 vorgesehen, um die gefaltete Konfiguration lösbar zu fixieren. In entsprechender Weise wird der in den Figuren 5 rechte Seitenabschnitt 26 gefaltet, so dass die in Figur 5e dargestellte Konfiguration erhalten wird.

Sodann sind in Figur 5e zwei in Querrichtung verlaufende Faltachsen 82 dargestellt, um welche die schon vorgefaltete Behandlungsstuhlabdeckung dann gewissermaßen von unten nach oben einrollend umgefaltet wird, so dass die in Figur 5f dargestellte Konfiguration erhalten wird. Schließlich erfolgt eine Faltung um eine weitere in Querrichtung 44 verlaufende Faltachse 84, so dass die in Figur 5g dargestellte Konfiguration erhalten wird. Daran anschließend wird vorzugsweise um eine weitere in Längsrichtung 50 erstreckte Faltachse 86 gefaltet, so dass die in Figur 5h dargestellte kompakt gefaltete Konfiguration für die Abgabe an den Handel erreicht wird.

Figuren 6a bis c skizzieren eine einstückige Herstellung der Behandlungsstuhlabdeckung 20 aus einer einzigen Flachmaterialbahn 90 in materialsparender Weise.

Figuren 7a bis c verdeutlichen die zusätzliche Ausbildung einer Eingriffsfalte als manuell ergreifbare Handhabungshilfe zum Überstülpen der Behandlungsstuhlabdeckung 20 über die Rückenlehne eines Behandlungsstuhls, und zwar mit Blick auf die gegenüberliegende Seite verglichen etwa mit Figur 4a. Die Eingriffsfalte 92 ist im Bereich der Tasche 28 ausgebildet, und zwar durch Umfalten eines der Öffnung der Tasche nächstgelegenen Randabschnitts 94. Der Umschlag und die randseitige Fixierung ist aus den Schnittansichten der Figuren 7b und 7c ersichtlich.

Anhand Figur 8 wird ein weiteres bevorzugtes Merkmal der vorliegenden Erfindung erläutert, wobei die Blickrichtung entsprechend Figur 7a von hinten gewählt ist. Die übereinander zu liegen kommenden Teilabschnitte 30, 32 der jeweiligen Seitenabschnitte 26 sind in Querrichtung 44 nicht gleich groß und damit in der dargestellten Konfiguration nicht kongruent. Der beim späteren Gebrauch äußere Teilabschnitt 30, der also nicht entlang der Fügelinie 42 an den ersten und gegebenenfalls zweiten Abschnitt angefügt ist, ist in Querrichtung 44 kürzer erstreckt als der andere Teilabschnitt 32, der entlang der Fügelinie 42 an den ersten und zweiten Abschnitt 24 angefügt ist. Dies erleichtert gerade die Ausbildung dieser Fügeverbindung entlang der Fügelinie 42, da durch die Querbeabstandung 96 prozesssicher gewährleistet werden kann, dass der spätere äußere Teilabschnitt 30 nicht von der Fügeverbindung erfasst wird.

Figur 8 verdeutlicht ein weiteres bevorzugtes Detailmerkmal der erfindungsgemäßen Behandlungsstuhlabdeckung, wonach der stirnseitige Rand 34 der Seitenabschnitte nicht kantenbündig mit einem vorderen stirnseitigen Rand 98 des der Sitzfläche zugeordneten ersten Abschnitts 22 verläuft, sondern der erste Abschnitt 22 erstreckt sich in Längsrichtung 50 weiter nach vorn. Hierdurch entsteht ein Überstand, der auch in Form eines zusätzlich angefügten Materialabschnitts 99 ausgebildet sein kann und in vorteilhafter Weise für eine noch weitergehende Abdeckung des unteren Bereichs des Behandlungsstuhls 2 dient.

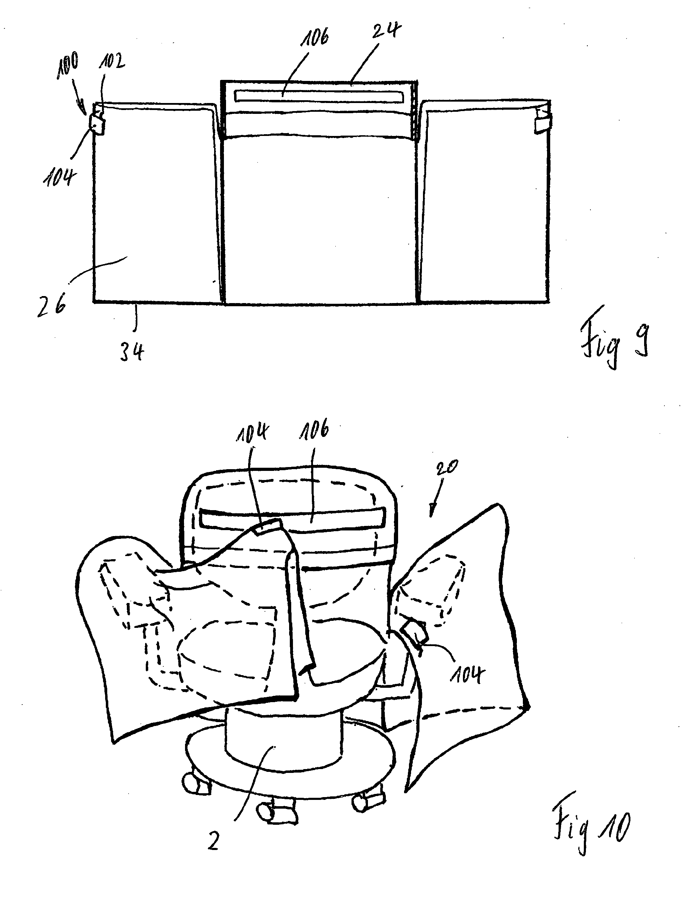

Schließlich verdeutlichen die Figuren 9 und 10 ein weiteres bevorzugtes Detailmerkmal der vorliegenden Erfindung. Danach sind lösbar haftend wirkende Fixiermittel 100 vorgesehen, und zwar vorzugsweise in einem der vorderen geschlossenen Stirnseite 34 gegenüberliegenden hinteren Bereich 102 der Seitenabschnitte 26. Diese Fixiermittel 100 umfassen eine erste Fixierkomponente 104, beispielsweise ein hakenbildendes Material, welche mit einer zweiten Fixierkomponente 106 zusammenwirkt, die auf der Rückseite des zweiten der Rückenlehne zugeordneten Abschnitts 24, beispielhaft und vorzugsweise in Form eines in Querrichtung 44 erstreckten Streifens vorgesehen ist. Diese zweite Fixierkomponente 106 kann beispielsweise von einem schlaufenbildenden Material gebildet sein; es kann sich hierbei beispielsweise auch um eine textil anmutende Außenseite des den zweiten Abschnitt 24 bildenden Flachmaterials handeln. Figur 10 zeigt wie die Behandlungsstuhlabdeckung im auf den Behandlungsstuhl 2 aufgezogenen Zustand lösbar unter Verwendung der ersten und zweiten Fixierkomponenten 104 und 106 fixiert werden kann.

## Patentansprüche

1. Wegwerfbare Behandlungsstuhlabdeckung (20) für den einmaligen Gebrauch für einen Behandlungsstuhl (2) mit einer Sitzfläche (6), einer Rückenlehne (8) und zwei seitlichen Armauflagen (10) im medizinischen Bereich, insbesondere im Operationssaal, wobei die Abdeckung (20) aus flächenhaften Abschnitten eines Flachmaterials gefertigt ist und herstellerseitig in eine gefaltete, zum bestimmungsgemäßen Gebrauch entfaltbare Konfiguration gebracht ist, wobei die Abdeckung (20) einen ersten der Sitzfläche (6) zugeordneten Abschnitt (22), einen zweiten der Rückenlehne (8) zugeordneten Abschnitt (24), und zwei den Armauflagen (10) zugeordnete Seitenabschnitte (26) aufweist, die jeweils spielbehaftet eine Armauflage (10) überfangen, indem sie eine U-Form bilden, die an einer vorderen Stirnseite (34) entlang einer Fügelinie (36) geschlossen ist und an einer gegenüberliegenden hinteren Seite und an einer unteren Seite offen ist, **dadurch gekennzeichnet, dass** die Abdeckung (20) aus flächenhaften zusammenhängenden Abschnitten (22, 24, 26) des Flachmaterials gefertigt ist und dass ein jeweiliger Seitenabschnitt (26) auf die spätere Sichtseite des ersten der Sitzfläche zugeordneten Abschnitts (22) und je nach Länge gegebenenfalls auch auf die spätere Sichtseite des zweiten der Rückenlehne zugeordneten Abschnitts (24) eingeschlagen ist.

2. Behandlungsstuhlabdeckung nach Anspruch 1, **gekennzeichnet durch** eine von dem zweiten Abschnitt (24), insbesondere **durch** Umfalten des zweiten Abschnitts auf sich selbst gebildete Tasche (28), die über die Rückenlehne (8) des Behandlungsstuhls (2) stülpbar ist.

3. Behandlungsstuhlabdeckung nach Anspruch 1 oder 2, **dadurch gekennzeichnet, dass** im Bereich des zweiten der Rückenlehne zugeordneten Abschnitts (24), insbesondere zusätzlich zu einer Tasche (28), eine Eingriffsfalte (92) oder ein zusätzliches manuell ergreifbares Mittel als Handhabungshilfe beim Aufziehen der Behandlungsstuhlabdeckung auf den Behandlungsstuhl (2) vorgesehen ist.

4. Behandlungsstuhlabdeckung nach Anspruch 1, 2 oder 3, **dadurch gekennzeichnet, dass** die Seitenabschnitte (26) als separate Abschnitte an den ersten der Sitzfläche (6) zugeordneten Abschnitt (22) der Abdeckung angefügt sind.

5. Behandlungsstuhlabdeckung nach einem oder mehreren der vorstehenden Ansprüche, **dadurch gekennzeichnet, dass** die Seitenabschnitte (26) in einem der vorderen Stirnseite (34) gegenüberliegenden hinteren Bereich nicht an den zweiten der Rückenlehne (8) zugeordneten Abschnitt (24) oder an den ersten der Sitzfläche (6) zugeordneten Abschnitt (22) angebunden sind, insbesondere dass die Seitenabschnitte (26) über wenigstens 7,5 cm, weiter insbesondere über wenigstens 10 cm, weiter insbesondere über wenigstens 15 cm, aber insbesondere über höchstens 30 cm, weiter insbesondere über höchstens 20 cm Längserstreckung nicht an den zweiten der Rückenlehne (8) zugeordneten Abschnitt (24) oder an den ersten der Sitzfläche (6) zugeordneten Abschnitt (22) angebunden sind.

6. Behandlungsstuhlabdeckung nach einem oder mehreren der vorstehenden Ansprüche, **dadurch gekennzeichnet, dass** der erste Abschnitt (22) und der zweite Abschnitt (24) einstückig ineinander übergehen.

7. Behandlungsstuhlabdeckung nach einem oder mehreren der vorstehenden Ansprüche, **dadurch gekennzeichnet, dass** die vordere Stirnseite (34) des jeweiligen Seitenabschnitts (26) und die vordere Kante des ersten Abschnitts (22) versetzt angeordnet sind, derart dass der erste Abschnitt (22) in Längsrichtung (50) vorsteht, insbesondere indem dort ein zusätzlicher Abschnitt (99) angefügt ist.

8. Behandlungsstuhlabdeckung nach einem oder mehreren der vorstehenden Ansprüche, **dadurch gekennzeichnet, dass** in einem direkt oberhalb der Armauflagen (10) zu liegen kommenden Bereich der Seitenabschnitte (26) keine Materialübergänge, Fügelinien oder -nähte oder sonstige Unstetigkeiten vorgesehen sind.

9. Behandlungsstuhlabdeckung nach einem oder mehreren der vorstehenden Ansprüche, **dadurch gekennzeichnet, dass** ein jeweiliger Seitenabschnitt (26) dadurch gebildet ist, dass ein Flachmaterialabschnitt um eine in der Längsrichtung (50) verlaufende Achse (52) auf sich selbst gefaltet ist und die aufeinander gefalteten Teilabschnitte (30, 32) entlang eines die vordere Stirnseite (34) der Abdeckung bildenden und in der Querrichtung (44) verlaufenden Rands (54) miteinander gefügt sind und dass ein in der Längsrichtung (50) verlaufender Rand nur eines der aufeinander gefalteten Teilabschnitte (32) mit dem ersten der Sitzfläche zugeordneten Abschnitt (22) gefügt ist.

10. Behandlungsstuhlabdeckung nach einem oder mehreren der vorstehenden Ansprüche, **gekennzeichnet durch** lösbar haftend wirkende Fixiermittel (100), um die Behandlungsstuhlabdeckung im drapierten auf den Behandlungsstuhl aufgezogenen Zustand lösbar zu fixieren.

11. Behandlungsstuhlabdeckung nach Anspruch 10, **dadurch gekennzeichnet, dass** die Seitenabschnitte (26) in einem der vorderen geschlossenen Stirnseite (34) gegenüberliegenden hinteren Bereich eine erste Fixierkomponente (104) aufweisen, die mit einer zweiten Fixierkomponente (106) im Bereich des zweiten der Rückenlehne zugeordneten Abschnitts (24) lösbar haftend zusammenwirkt, wobei diese zweite Fixierkomponente (106) insbesondere streifenförmig ausgebildet ist.

12. Behandlungsstuhlabdeckung nach einem oder mehreren der vorstehenden Ansprüche, **dadurch gekennzeichnet, dass** bei einem jeweiligen Seitenabschnitt (26) eine Umfaltung (60) um eine in der Querrichtung (44) verlaufende Achse auf sich selbst vorgesehen ist, insbesondere die Umfaltung wenigstens 5 cm, weiter insbesondere wenigstens 7,5 cm, weiter insbesondere wenigstens 10 cm, weiter insbesondere höchstens 30 cm, weitere insbesondere höchstens 25 cm, weiter insbesondere höchstens 20 cm tief ist.

13. Behandlungsstuhlabdeckung nach einem oder mehreren der vorstehenden Ansprüche, **dadurch gekennzeichnet, dass** ein jeweiliger Seitenabschnitt (26) um eine diagonal, also schräg zur Längsrichtung (50) und zur Querrichtung (44) verlaufende Faltachse (64) auf sich selbst nach innen gefaltet ist.

14. Behandlungsstuhlabdeckung nach Anspruch 12 und 13, **dadurch gekennzeichnet, dass** die diagonale Faltung derart ist, dass der gefaltete Bereich unter die Umfaltung (60) erstreckt ist.

15. Behandlungsstuhlabdeckung nach einem oder mehreren der vorstehenden Ansprüche, **dadurch gekennzeichnet, dass** ein jeweiliger Seitenabschnitt (26) um eine im Bereich der Anfügung der Seitenabschnitte (26) an den ersten Abschnitt (22) liegende Achse (68) auf die spätere Sichtseite des ersten der Sitzfläche zugeordneten Abschnitts (22) eingeschlagen ist.

16. Behandlungsstuhlabdeckung nach Anspruch 13, 14 oder 15, **dadurch gekennzeichnet, dass** der jeweilige diagonal gefaltete Seitenabschnitt (26) und/oder der jeweilige auf die spätere Sichtseite des ersten der Sitzfläche zugeordneten Abschnitts (22) eingeschlagene Seitenabschnitt (26) durch manuell lösbare Fixiermittel (66, 69), insbesondere in Form von Haftklebemitteln, insbesondere mit Sollbruchlinien, in der gefalteten Konfiguration gehalten ist.

17. Behandlungsstuhlabdeckung nach Anspruch 15 oder 16, **dadurch gekennzeichnet, dass** sie um wenigstens drei in Querrichtung (44) verlaufende Faltachsen (70, 72, 82, 84) auf sich selbst gefaltet ist, und zwar vorzugsweise derart, dass ein in Längsrichtung (50) endseitiger Teilabschnitt (74) des zweiten der Rückenlehne zugeordneten Abschnitts (24) oben zu liegen kommt.

18. Behandlungsstuhlabdeckung nach Anspruch 17, **dadurch gekennzeichnet, dass** sie um wenigstens zwei in Querrichtung (44) verlaufende Faltachsen (72) Z-förmig auf sich selbst gefaltet ist.

19. Behandlungsstuhlabdeckung nach einem oder mehreren der vorstehenden Ansprüche, **dadurch gekennzeichnet, dass** sie insbesondere nach dem Falten um die in Querrichtung (44) verlaufenden Achsen (70, 72, 82, 84) einmal oder mehrmals um in Längsrichtung (50) verlaufende Faltachsen (76, 78, 86) auf sich selbst gefaltet ist.

20. Behandlungsstuhlabdeckung nach einem oder mehreren der vorstehenden Ansprüche, **dadurch gekennzeichnet, dass** die Seitenabschnitte (26), insbesondere nach dem Einschlagen auf die spätere Sichtseite des ersten der Sitzfläche zugeordneten Abschnitts (22), um wenigstens eine in Längsrichtung (50) verlaufende Faltachse (80, 81) auf sich selbst gefaltet ist.

21. Behandlungsstuhlabdeckung nach Anspruch 20, **dadurch gekennzeichnet, dass** die wenigstens eine Faltung um die wenigstens eine in Längsrichtung (50) verlaufende Faltachse (80) auch den ersten und zweiten Abschnitt (22, 24) erfasst.

## Claims

1. A disposable treatment chair covering (20) for one-time use for a treatment chair (2), having a seat surface (6), a backrest (8) and two lateral arrests (10), in the medical field, in particular in the operating room, the covering (20) being made from two-dimensional portions of a flat material and put by the manufacturer into a folded configuration that can be unfolded for the intended use, and the covering (20) having a first portion (22) associated with the seating surface (6), a second portion (24) associated with the backrest (8), and two lateral portions (26) associated with the armrests (10), each of the lateral portions fitting with play over an armrest (10) by forming a U shape, which is closed on a front face end (34) along a joining line (36) and is open on an opposite, rear side and on a lower side, **characterized in that** the covering (20) is made from two-dimensional cohesive portions (22, 24, 26) of the flat material; and that each lateral portion (26) is folded in onto what is later the visible side of the first portion (22) associated with the seating surface and, depending on length, optionally also onto what is later the visible side of the second portion (24) associated with the backrest.

2. The treatment chair covering of claim 1, **characterized by** a pocket (28), formed from the second portion (24), in particular by folding over the second portion onto itself, which pocket can be fitted over the backrest (8) of the treatment chair (2).

3. The treatment chair covering of claim 1 or 2, **characterized in that** in the region of the second portion (24) associated with the backrest, in particular in addition to a pocket (28), a grasping fold (92) or an additional manually graspable means is provided as an aid in manipulation when putting the treatment chair covering onto the treatment chair (2).

4. The treatment chair covering of claim 1, 2 or 3, **characterized in that** the lateral portions (26), as separate portions, are joined to the first portion (22) of the covering that is associated with the seating surface (6).

5. The treatment chair covering of one or more of the foregoing claims, **characterized in that** the lateral portions (26), in a rear region opposite the front face end (34), are not bound to the second portion (24), associated with the backrest (8), or to the first portion (22), associated with the seating surface (6), and in particular that the lateral portions (26), along a distance of at least 7.5 cm, and furthermore in particular along a distance of at least 10 cm, and furthermore in particular along a distance of at least 15 cm, but in particular along a distance of at most 30 cm, and furthermore in particular at most 20 cm lengthwise are not bound to the second portion (24) associated with the backrest (8) or to the first portion (22) associated with the seating surface (6).

6. The treatment chair covering of one or more of the foregoing claims, **characterized in that** the first portion (22) and the second portion (24) are in one piece with one another.

7. The treatment chair covering of one or more of the foregoing claims, **characterized in that** the front face end (34) of each lateral portion (26) and the front edge of the first portion (22) are located in offset fashion, such that the first portion (22) protrudes in the longitudinal direction (50), in particular **in that** an additional portion (99) is attached there.

8. The treatment chair covering of one or more of the foregoing claims, **characterized in that** in a region of the lateral portions (26) that comes to rest directly above the armrests (10), no transitions of material, joining lines or seams or other discontinuities are provided.

9. The treatment chair covering of one or more of the foregoing claims, **characterized in that** each lateral portion (26) is formed **in that** a flat material portion is folded over onto itself in the axis (52) extending in the longitudinal direction (50), and the subsidiary portions (30, 32) folded onto one another are joined to one another along an edge (54) forming the front face end (34) of the covering and extending in the transverse direction (44); and that an edge, extending in the longitudinal direction (50), of only one of the subsidiary portions (32) folded onto one another is joined to the first portion (22) associated with the seating surface.

10. The treatment chair covering of one or more of the foregoing claims, **characterized by** fixation means (100) acting in detachably adhering fashion, in order to fix the treatment chair covering detachably, in the put-on state in which it is draped onto the treatment chair.

11. The treatment chair covering of claim 10, **characterized in that** the lateral portions (26), in a rear region opposite the front, closed face end (34), have first fixation component (104), which cooperates in detachably adhering fashion with a second fixation component (106) in the region of the second portion (24) associated with the backrest, and this second fixation component (106) is embodied in particular in striplike fashion.

12. The treatment chair covering of one or more of the foregoing claims, **characterized in that** in each lateral portion (26), a foldover (60) onto itself about an axis extending in the transverse direction (44) is provided; in particular, the foldover is at least 5 cm, furthermore in particular at least 7.5 cm, furthermore in particular at least 10 cm, furthermore in particular at most 30 cm, furthermore in particular at most 25 cm, and furthermore in particular at most 20 cm deep.

13. The treatment chair covering of one or more of the foregoing claims, **characterized in that** each lateral portion (26) is folded inward onto itself about a diagonal fold axis (64), that is, an axis extending obliquely to the longitudinal direction (50) and to the transverse direction (44).

14. The treatment chair covering of claims 12 and 13, **characterized in that** the diagonal folding is such that the folded region extends underneath the foldover (60).

15. The treatment chair covering of one or more of the foregoing claims, **characterized in that** each lateral portion (26) is folded in onto what is later the visible side of the first portion (22) associated with the seating face, about an axis (68) located in the region of the attachment of the lateral portions (26) to the first portion (22).

16. The treatment chair covering of claim 13, 14 or 15, **characterized in that** each diagonally folded lateral portion (26) and/or each lateral portion (26), folded in onto what is later the visible side of the first portion (22), associated with the seating surface, is retained in the folded configuration by manually detachable fixation means (66, 69), in particular in the form of adhesive means, in particular with rated breaking lines.

17. The treatment chair covering of claim 15 or 16, **characterized in that** it is folded onto itself about at least three fold axes (70, 72, 82, 84) extending in the transverse direction (44), specifically being folded preferably such that a subsidiary portion (74), on the end in the longitudinal direction (50), of the second portion (24) associated with the backrest comes to rest on the top.

18. The treatment chair covering of claim 17, **characterized in that** it is folded onto itself in Z-fashion about at least two fold axes (72) extending in the transverse direction (44).

19. The treatment chair covering of one or more of the foregoing claims, **characterized in that**, in particular after the folding about the axes (70, 72, 82, 84) extending in the transverse direction (44), it is folded onto itself once or multiple times about fold axes (76, 78, 86) extending in the longitudinal direction (50).

20. The treatment chair covering of one or more of the foregoing claims, **characterized in that** the lateral portions (26), in particular after being folded in onto what is later the visible side of the first portion (22) associated with the seating surface is folded over onto itself about at least one fold axis (80, 81) extending in the longitudinal direction (50).

21. The treatment chair covering of claim 20, **characterized in that** the at least one fold about the at least one fold axis (80) extending in the longitudinal direction (50) also involves the first and second portions (22, 24).

## Revendications

1. Couverture de chaise de soins (20) jetable pour l'usage unique pour une chaise de soins (2) comprenant une surface assise (6), un dossier (8) et deux appuie-bras (10) latéraux, dans le domaine médical, en particulier dans la salle d'opération, ladite couverture (20) étant réalisée à partir de portions en nappe d'une matière plate et étant mise, par le fabricant, dans une configuration pliée apte à être dépliée pour l'usage conforme à sa destination, ladite couverture (20) comprenant une première portion (22) associée à la surface assise (6), une deuxième portion (24) associée au dossier (8) et deux portions latérales (26) associées aux appuie-bras (10) qui, tout en présentant du jeu, s'étendent chacune sur un appuie-bras (10) en formant une forme d'U qui est fermée sur une face frontale avant (34) le long d'une ligne d'aille (36) et est ouverte sur une face arrière opposée et sur une face inférieure, **caractérisée par le fait que** ladite couverture (20) est réalisée à partir de portions (22, 24, 26) en nappe continues de la matière plate et qu'une portion latérale (26) respective est repliée sur la surface visible ultérieurement de ladite première portion (22) associée à la surface assise et, selon la longueur, le cas échéant également sur la surface visible ultérieurement de ladite deuxième portion (24) associée au dossier.

2. Couverture de chaise de soins selon la revendication 1, **caractérisée par** une poche (28) qui est formée par ladite deuxième portion (24), en particulier en rabattant ladite deuxième portion sur elle-même, et qui peut être mise sur le dossier (8) de la chaise de soins (2).

3. Couverture de chaise de soins selon la revendication 1 ou 2, **caractérisée par le fait qu'**un pli de préhension (92) ou un moyen supplémentaire apte à être saisi manuellement est prévu au niveau de ladite deuxième portion (24) associée au dossier, en particulier en plus d'une poche (28), comme aide de manipulation lorsque la couverture de chaise de soins est posée sur la chaise de soins (2).

4. Couverture de chaise de soins selon la revendication 1, 2 ou 3, **caractérisée par le fait que** les portions latérales (26) sont accolées en tant que portions séparées à ladite première portion (22) de la couverture, qui est associée à la surface assise (6).

5. Couverture de chaise de soins selon l'une ou plusieurs des revendications précédentes, **caractérisée par le fait que** les portions latérales (26) ne sont pas attachées à la deuxième portion (24) associée au dossier (8) ou à la première portion (22) associée à la surface assise (6), dans une zone arrière située en regard de la face frontale avant (34), que, en particulier, les portions latérales (26) ne sont pas attachées à la deuxième portion (24) associée au dossier (8) ou à la première portion (22) associée à la surface assise (6), sur une extension longitudinale de 7,5 cm au moins, encore en particulier de 10 cm au moins, encore en particulier de 15 cm au moins, mais en particulier de 30 cm tout au plus, encore en particulier de 20 cm tout au plus.

6. Couverture de chaise de soins selon l'une ou plusieurs des revendications précédentes, **caractérisée par le fait que** la première portion (22) et la deuxième portion (24) se confondent en une seule pièce.

7. Couverture de chaise de soins selon l'une ou plusieurs des revendications précédentes, **caractérisée par le fait que** ladite face frontale avant (34) de la portion latérale (26) respective et l'arête avant de la première portion (22) sont disposées de manière décalée de telle sorte que ladite première portion (22) fait saille dans la direction longitudinale (50), en particulier en y rapportant une portion (99) supplémentaire.

8. Couverture de chaise de soins selon l'une ou plusieurs des revendications précédentes, **caractérisée par le fait que**, dans une zone des portions latérales (26) qui vient se positionner directement au-dessus des appuie-bras (10), on ne prévoit pas de transitions de matière, de lignes ou coutures d'aille ou d'autres discontinuités.

9. Couverture de chaise de soins selon l'une ou plusieurs des revendications précédentes, **caractérisée par le fait qu'**une portion latérale (26) respective est formée **par le fait qu'**une portion de matière plate est pliée sur elle-même sur un axe (52) s'étendant dans la direction longitudinale (50) et que les portions partielles (30, 32) pliées l'une sur l'autre sont jointes entre elles le long d'un bord (54) formant ladite face frontale avant (34) de la couverture et s'étendant dans la direction transversale (44), et qu'un bord s'étendant dans la direction longitudinale (50), de l'une seulement des portions partielles (32) pliées l'une sur l'autre est joint avec ladite première portion (22) associée à la surface assise.

10. Couverture de chaise de soins selon l'une ou plusieurs des revendications précédentes, **caractérisée par** des moyens de fixation (100) agissant par adhérence amovible afin de fixer d'une manière amovible ladite couverture de chaise de soins en état drapé posé sur la chaise de soins.

11. Couverture de chaise de soins selon la revendication 10, **caractérisée par le fait que**, dans une zone arrière située en regard de la face frontale avant (34) fermée, lesdites portions latérales (26) présentent un premier composant de fixation (104) qui coopère par adhérence amovible avec un deuxième composant de fixation (106) au niveau de ladite deuxième portion (24) associée au dossier, ce deuxième composant de fixation (106) étant réalisé en particulier en forme de bande.

12. Couverture de chaise de soins selon l'une ou plusieurs des revendications précédentes, **caractérisée par le fait que**, dans une portion latérale (26) respective, on prévoit un rabat (60) sur elle-même, sur un axe s'étendant dans la direction transversale (44), que, en particulier, ledit rabat présente une profondeur de 5 cm au moins, encore en particulier de 7,5 cm au moins, encore en particulier de 10 cm au moins, encore en particulier de 30 cm tout au plus, encore en particulier de 25 cm tout au plus, encore en particulier de 20 cm tout au plus.

13. Couverture de chaise de soins selon l'une ou plusieurs des revendications précédentes, **caractérisée par le fait qu'**une portion latérale (26) respective est pliée vers l'intérieur sur elle-même sur un axe de pliage (64) s'étendant en diagonal, donc en biais, par rapport à la direction longitudinale (50) et par rapport à la direction transversale (44).

14. Couverture de chaise de soins selon la revendication 12 et 13, **caractérisée par le fait que** le pliage diagonal est tel que la zone pliée s'étend sous ledit rabat (60).

15. Couverture de chaise de soins selon l'une ou plusieurs des revendications précédentes, **caractérisée par le fait qu'**une portion latérale (26) respective est repliée sur un axe (68) situé au niveau de la jonction des portions latérales (26) à la première portion (22), sur la surface visible ultérieurement de la première portion (22) associée à la surface assise.

16. Couverture de chaise de soins selon la revendication 13, 14 ou 15, **caractérisée par le fait que** la portion latérale (26) respective pliée en diagonal et/ou la portion latérale (26) respective repliée sur la surface visible ultérieurement de la première portion (22) associée à la surface assise est maintenue dans la configuration pliée par des moyens de fixation (66, 69) amovibles manuellement, en particulier sous forme de moyens adhésifs de contact, en particulier avec des lignes destinées à la rupture.

17. Couverture de chaise de soins selon la revendication 15 ou 16, **caractérisée par le fait qu'**elle est pliée sur elle-même sur au moins trois axes de pliage (70, 72, 82, 84) s'étendant dans la direction transversale (44), à savoir de préférence de telle sorte qu'une portion partielle (74) terminale dans la direction longitudinale (50), de ladite deuxième portion (24) associée au dossier vient se placer en haut.

18. Couverture de chaise de soins selon la revendication 17, **caractérisée par le fait qu'**elle est pliée en Z sur elle-même sur au moins deux axes de pliage (72) s'étendant dans la direction transversale (44).

19. Couverture de chaise de soins selon l'une ou plusieurs des revendications précédentes, **caractérisée par le fait que**, en particulier après le pliage sur les axes (70, 72, 82, 84) s'étendant dans la direction transversale (44), elle est pliée une ou plusieurs fois sur elle-même sur des axes de pliage (76, 78, 86) s'étendant dans la direction longitudinale (50).

20. Couverture de chaise de soins selon l'une ou plusieurs des revendications précédentes, **caractérisée par le fait que** lesdites portions latérales (26), en particulier après avoir été repliées sur la surface visible ultérieurement de ladite première portion (22) associée à la surface assise, sont pliées sur elles-mêmes sur au moins un axe de pliage (80, 81) s'étendant dans la direction longitudinale (50).

21. Couverture de chaise de soins selon la revendication 20, **caractérisée par le fait que** ledit au moins un pliage sur ledit au moins un axe de pliage (80) s'étendant dans la direction longitudinale (50) atteint également lesdites première et deuxième portions (22, 24).
